# EUROPEAN PATENT APPLICATION

(11) **EP 3 828 286 A1**
(43) Date of publication of application: **02.06.2021**
(21) Application number: 19837440.7
(22) Date of filing: 03.04.2019
(51) Int. Cl.: C12Q 1/6883, C12N 15/11

(54) **SET OF PATHOGENIC GENES FOR MENTAL RETARDATION AND ADVANCED COGNITIVE IMPAIRMENT AND DETECTION PRIMER AND KIT THEREFOR**

(30) Priority: 17.07.2018 CN 201810780632
(71) Applicant: The First Affiliated Hospital of Dalian Medical University, Dalian, Liaoning 116011 (CN)
(72) Inventor: LIU, Jing, Dalian, Liaoning 116011 (CN); WANG, Liang, Dalian, Liaoning 116011 (CN); XIN, Chengqi, Dalian, Liaoning 116011 (CN); MA, Jingyun, Dalian, Liaoning 116011 (CN)
(74) Representative: Proi World Intellectual Property GmbH
(86) International application number: PCT/CN2019/081210
(87) International publication number: WO 2020/015387

(57) **Abstract**

Disclosed are a set of pathogenic genes of neuropsychiatric retardation and advanced cognitive disorder, detection primer and kit thereof. The present disclosure uses the strategy of combining the whole-genome sequencing and the next-generation of optical mapping technology to determine the mutation sites of SLC16A2, KIF4A, MED15 and FAM83G and five structural variant genes (CT55, DTX2P1-UPK3BP1-PMS2P11, EBF2, FAM173B, FAM173B and TMTC1). The present disclosure obtains the pathogenic genes of neuropsychiatric retardation and advanced cognitive disorder. The detection of a mutation in said genes can assist in the diagnosis of neuropsychiatric retardation and advanced cognitive disorder.

## Description

### Technical Field

The present invention belongs to the field of biomedicine and gene detection, and relates to a set of pathogenic genes of neuropsychiatric retardation and advanced cognitive disorder and detection primer and kit thereof.

### Background Art

The basis of neuropsychiatric development is the growth and development of the nervous system and the neuropsychiatric activity is the performance of the nervous system's response to internal and external stimuli, including perception, reflection, movement, language, and emotional response to people around, etc. Neuropsychiatric retardation is a common developmental disorder in children, with the main clinical manifestations of growth retardation, mental retardation, ataxia, athetosis, facial deformity, contracture of joint, cognitive disorder, dysarthria, epileptic seizure, etc.

There are many diseases that can cause neuropsychiatric retardation and advanced cognitive disorder, including Allen-Hernden-Dudley syndrome (AHDS), cerebral palsy (CP), hereditary spastic paraplegia (HSP), etc., which has the characteristics of familial and lifelong nature, and has extremely high rate of disability and foolishness, having great harm. The inheritance mode of these diseases mainly includes single gene inheritance, polygene inheritance and chromosomal variation, etc. However, the etiology and pathogenesis of such diseases have not yet been clarified; especially the genetic pathogenic factors are urgently to further studied and discussed.

### Summary of the Invention

The present invention selected a family similar to AHDS with mixed CP (including a sick elder brother and two sick identical twin brothers, whose parents have normal phenotypes) as the research object, whose clinical symptoms are neuropsychiatric retardation and advanced cognitive disorder. The genetic pathogenic factors of the family are studied in the whole genome by a strategy of combining whole-genome high-throughput sequencing (WGS) and next-generation optical mapping (NGM) technology, to identify a new pathogenic mutation site of SLC16A2, mutation sites of KIF4A, MED15, FAM83G, and 5 structural variant genes (CT55, DTX2P1-UPK3BP1-PMS2P11, EBF2, FAM173B and TMTC1). At present, there are no reports that the mutation sites and structural variations of these genes lead to neuropsychiatric retardation and advanced cognitive disorder.

One aspect of the present invention is to provide a set of pathogenic genes of neuropsychiatric retardation and advanced cognitive disorder.

Further, the present invention relates to a set of pathogenic genes of neuropsychiatric retardation and advanced cognitive disorder, including mutation sites of SLC16A2, KIF4A, MED15 and FAM83G, and 5 structural variant genes (CT55, DTX2P1-UPK3BP1-PMS2P11, EBF2, FAM173B, and TMTC1).

Further, in the present invention, a cDNA coding region sequence of the SLC16A2 mutant gene is that the nucleotide G at position 1357 in exon region 5 of SLC16A2 gene sequence mutates into nucleotide C, c.1357 G>C (p.G453R).

Further, in the present invention, a cDNA coding region sequence of the KIF4A mutant gene is that the nucleotide C at position 1472 in exon region 14 of KIF4A gene sequence mutates into nucleotide T, 1472 C >T (p.A491V).

Further, in the present invention, a cDNA coding region sequence of the MED 15 mutant gene is that CAG nucleotides are inserted at position 573 in exon region 6 of the MED15 gene sequence, c.573insCAG (p.191insQ).

Further, in the present invention, a cDNA coding region sequence of the FAM83G mutant gene is that ACC nucleotides are inserted at position 2455 in exon region 6 of the FAM83G gene sequence, c.2455insACC (p.819insH).

Further, in the present invention, the five structural variant genes are CT55, DTX2P1-UPK3BP1-PMS2P11, EBF2, FAM173B, and TMTC1, with the specific information shown in the following table:

| Inheritance mode | Structural variation | Starting position | Ending position | Covering gene |
|---|---|---|---|---|
| X-linked inheritance | Insertion | 134294230 | 134346153 | CT55 |
| Autosomal recessive inheritance | Insertion | 76621574 | 76629524 | DTX2P1-UPK3BP1-PMS2P11 |
| | Insertion | 25862704 | 25867245 | EBF2 |
| | Deletion | 10227256 | 10232232 | FAM173B |
| | Deletion | 29839167 | 29850639 | TMTC1 |

Another aspect of the present invention is to provide a detection primer for screening pathogenic genes of neuropsychiatric retardation and advanced cognitive disorder, comprising a PCR primer designed for mutant gene sequences in the set of pathogenic genes provided in the present invention; wherein the PCR primer sequence is selected from:
The reference sequence of the SLC16A2 gene involved in the present invention is showed in SEQ ID NO.1;
The primer sequence for detecting the mutation site of the SLC16A2 gene involved in the present invention is as follows:
   Forward primer: 5'-AAAGGAGATGTGATGCTATGTGG-3', SEQ ID NO. 5
   Reverse primer: 5'-TGATAAGGGGTTTCACGCACTCT-3', SEQ ID NO. 6
The reference sequence of the KIF4A gene involved in the present invention is showed in SEQ ID NO. 2;
The primer sequence for detecting the mutation site of the KIF4A gene involved in the present invention is as follows:
   Forward primer: 5'- ACTATTGGGGTTTCTGTAACTAT-3', SEQ ID NO.7
   Reverse primer: 5'-ACTAGCCTCTAACAAGAAATCAG-3', SEQ ID NO.8
The reference sequence of the MED15 gene involved in the present invention is showed in SEQ ID NO.3;
The primer sequence for detecting the mutation site of the MED 15 gene involved in the present invention is as follows:
   Forward primer: 5'-GAGACGGGGTTTCGCCAGGTTGT-3', SEQ ID NO.9
   Reverse primer: 5'- GCGGGAGTTGTGATGGTTGGTTC-3', SEQ ID NO. 10
The reference sequence of the FAM83G gene involved in the present invention is showed in SEQ ID NO. 4;
The primer sequence for detecting the mutation site of the FAM83G gene involved in the present invention is as follows:
   Forward primer: 5'-CCCTTCTTGCTCAGCCTCACTCT-3', SEQ ID NO.11
   Reverse primer: 5'- CTCCAGGACCATTGCCAACACCA-3', SEQ ID NO.12

One more aspect of the present invention is to provide a kit for detecting gene mutation sites of neuropsychiatric retardation and advanced cognitive disorder, comprising the PCR primer sequence provided above, PCR amplification enzyme, and PCR amplification buffer. Specifically, in some embodiments of the present invention, it is a PCR reaction system based on Takara-PrimeSTAR Max DNA Polymerase.

Beneficial effects: the present invention adopts the strategy of combining whole-genome high-throughput sequencing (WGS) and next-generation optical mapping (NGM) technology to study the gene mutations and structural variations in the whole genome of the family, in combination with bioinformatics analysis to identify a new mutation site of SLC16A2, mutation sites of KIF4A, MED15, FAM83G, and 5 structural variant genes, so as to provide a set of pathogenic genes of neuropsychiatric retardation and advanced cognitive disorder, and detection primer and a kit for detecting gene mutation sites.

### Brief Description of the Drawings

FIG. 1 is the family tree;
FIG. 2 is the verification results of Sanger sequencing.

### Detailed Description of Embodiments

The following non-limiting examples may enable those of ordinary skill in the art to more fully understand the present invention, but do not limit the present invention in any way.

### Embodiment 1

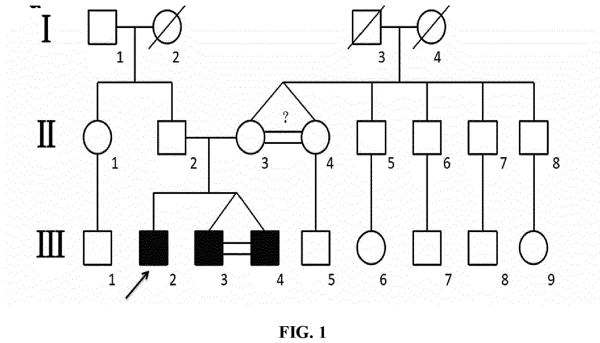

The present invention collected a family similar to AHDS with mixed CP, including a sick elder brother (III-2 in FIG. 1) and two sick identical twin brothers (III-3 and III-4), whose parents had normal phenotypes. The main clinical symptoms of the patients were neuropsychiatric retardation and advanced cognitive disorder.

Patient III-2, male, full-term natural labour at 42 weeks of gestation, with a birth weight of 4200 g; the cranial nerve examination was substantially normal; motor development retardation was manifested in the reduction of trunk and limb muscle strength. In resting state, muscle tension decreased; but when active activities and emotional tension, muscle tension would increase unevenly and even tremor, making him difficult to take care of himself in daily life (eating, toileting).

Patients III-3 and III-4, both males, identical twins, were delivered by cesarean section at 38 weeks of gestation, with birth weights of 2800 g and 2650 g respectively; the cranial nerve examinations were substantially normal; their muscle tensions were worse than that of patient III-2, and even obvious unstable posture and involuntary head swing.

All three patients presented with cup ear, slender narrow face; hypotonia, motor development retardation; angular salivation, fixed head and neck leaning, motor deficit unable to walk independently and may be accompanied by ataxia, dysarthria accompanied with aphasia; hands presented with spasticity or athetosis; serious impairment of intellectual cognitive function; behavioral activities tended to be passive, and less irritable and radicalness state; increased serum T3 and normal T4.

Based on the phenotypes of patients, it was preliminarily judged that all 3 patients suffered from neuropsychiatric retardation and advanced cognitive disorder. The present invention performed whole genome sequencing and large-segment structural variation analysis on patients (III-2, III3 and III-4) and their parents (II-2 and II-3) by a strategy of combining Illumina HiSeq X Ten sequencing and Bionano Saphyr next-generation optical mapping technology.

Due to the family characteristics and hereditary feature, the family was identified as X-linked recessive inheritance or autosomal recessive inheritance, and recessive homozygous mutations should be considered firstly. Therefore, the mutations met the following requirements were screened firstly: homozygous mutation shared by the three patients, and wild-type or heterozygous mutation in normal individual (including single nucleotide variations (SNVs), small indels and large structural variants (SVs)); then the variants with the smallest allele frequency greater than 5% in the Thousand Genome Project were filtered out; followed by the variants and synonymous mutations located in the intergenic region and the intron region were filtered out ; finally obtained a new mutation site of SLC16A2, mutation sites of KIF4A, MED15, FAM83G, and 5 structural mutation genes.

### Embodiment 2

### Sample Collection

According to the requirements of the World Medical Association "Declaration of Helsinki", the patients' parents signed an informed consent form with the Ethics Committee of the First Affiliated Hospital of Dalian Medical University.

### Sample Preparation

Peripheral venous blood of the patients (III-2, III3, and III4) and their parents (II-2 and II-3) was taken, from which the high-molecular-weight genomic DNA was extracted according to the Bionano Prep Blood DNA Isolation Protocol (Bionano Genomics Inc.) for high-throughput sequencing and next-generation optical mapping analysis respectively. The purity and concentration of the DNA was measured by a spectrophotometer, and the DNA quality was detected by agarose gel electrophoresis.

Whole-genome High-throughput Sequencing and the Next-generation Optical Mapping Analysis
1. Whole-genome sequencing was carried out in accordance with the instructions provided by Illumina for library construction and computer sequencing. The sequencing platform was the Illumina HiSeq X Ten sequencer. After quality filtering, the original data were aligned with the reference gene hg38 using BWA-MEM software, and the repetitive sequences generated by PCR were filtered out by Picard and Samtools, followed by determining the types of SNP and indel by Bcftools.
2. The next-generation of optical mapping analysis was carried out in accordance with the instructions provided by Bionano Genomics for DNA labeling, and single-molecule data generated by Saphyr was used for determination of genome assembly and structural variation. After quality filtering, the original data were used for genome assembly and analysis by Bionano Solve software.

### Sanger Sequencing Verification

### 1. DNA extraction

Peripheral blood of patients III-2, III-3 and III4 and other normal individuals II-1, II-2, II-3, II-4, II-5, III-1 and III-5 in the family was collected, from which genomic DNA of leukocytes was extracted by conventional phenol-chloroform method. The purity and concentration of the DNA were measured with spectrophotometer, and the quality of the DNA was detected by agarose gel electrophoresis. The genomic DNA of each sample obtained had OD260/OD280 of 1.8-2.0, with the concentration not less than 50 ng/µl.

### 2. Primer design and PCR reaction

(1) Specific primers were designed for the mutation sites of SLC16A2, KIF4A, MED15 and FAM83G by referring to the human genome sequence hg38, with sequences showed in the following table:

| Gene name | Forward primer sequence (5'-3') | Reverse primer sequence (5'-3') |
|---|---|---|
| SLC16A2 | AAAGGAGATGTGATGCTATGTGG | TGATAAGGGGTTTCACGCACTCT |
| KIF4A | ACTATTGGGGTTTCTGTAACTAT | ACTAGCCTCTAACAAGAAATCAG |
| MED15 | GAGACGGGGTTTCGCCAGGTTGT | GCGGGAGTTGTGATGGTTGGTTC |
| FAM83G | CCCTTCTTGCTCAGCCTCACTCT | CTCCAGGACCATTGCCAACACCA |

(2) The PCR system was as follows (based on Takara-PrimeSTAR Max DNA Polymerase):

**Reaction system: 50 µl**

| Reagent | Dosage | Final concentration |
|---|---|---|
| PrimeSTAR Max Premix (2X) | 25 µl | 1X |
| Forward primer (10 µM) | 1 µl | 0.2 µM |
| Reverse primer (10 µM) | 1 µl | 0.2 µM |
| Template DNA (50 ng/µl) | 1 µl | |
| ddH₂O | 22 µl | |

(3) PCR reaction conditions were as follows:
(98°C 10 s, 55°C 5 s, 72°C 5 s), a total of 40 cycles, holding at 4°C.

The PCR amplification products of all samples (patients and normal subjects in the family) were thus obtained.

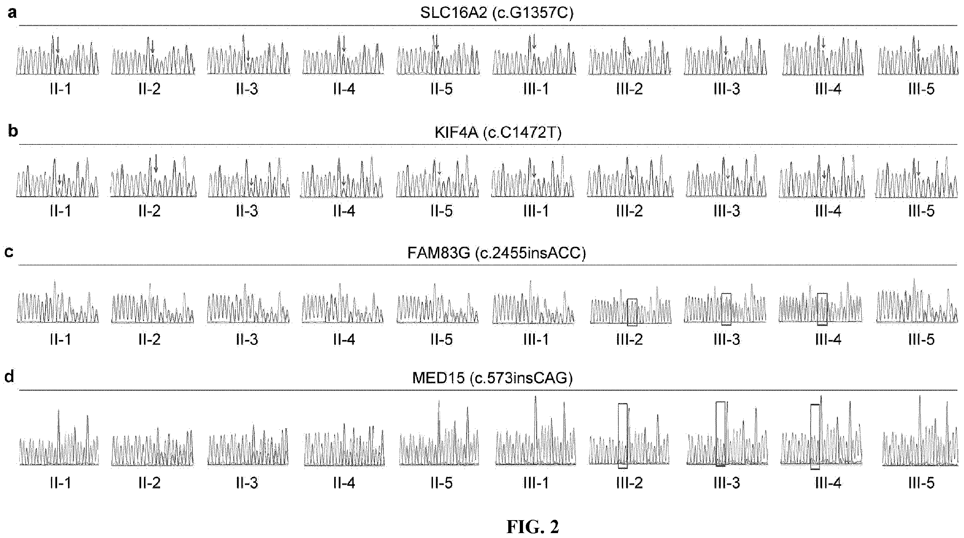

3. The relevant sequences of all genes were obtained from PCR products of all samples by the Sanger sequencing method, and the samples was classified as the wild type or the mutant type according to the sequencing results. Fig.2 shows the Sanger sequencing verification peak diagrams of 4 genes in all samples.

In summary, the verification of the mutation sites of the SLC16A2, KIF4A, MED15 and FAM83G genes in the family members of the patients found that the patients with SLC16A2 and KIF4A genes were all mutant types, while other normal subjects in the family were heterozygous mutations or wild-types; the patients with MED15 and FAM83G genes were all homozygous mutations, while other normal subjects in the family were heterozygous mutations or wild-types.

The present invention adopts the strategy of combining whole-genome high-throughput sequencing (WGS) and next-generation optical mapping (NGM) technology to study the gene mutations and structural variations in the whole genome of the family, in combination with bioinformatics analysis to identify a new mutation site of SLC16A2, mutation sites of KIF4A, MED15, FAM83G, and 5 structural variant genes, so as to provide pathogenic genes of neuropsychiatric retardation and advanced cognitive disorder and a kit for detecting gene mutation sites.

One aspect of the present invention is to provide a set of pathogenic genes of neuropsychiatric retardation and advanced cognitive disorder.

The present invention relates to a set of potential pathogenic genes of neuropsychiatric retardation and advanced cognitive disorder, including mutation sites of SLC16A2, KIF4A, MED15 and FAM83G, and 5 structural variant genes (CT55, DTX2P1-UPK3BP1-PMS2P11, EBF2, FAM173B and TMTC1).

The SLC16A2 mutation site involved in the present invention is c.1357 G>C (p.G453R).

The KIF4A mutation site involved in the present invention is c.1472 C> T (p.A491V).

The MED15 mutation site involved in the present invention is c.573insCAG (p.191insQ).

The FAM83G mutation site involved in the present invention is c.2455insACC (p.819insH).

The five structural variant genes involved in the present invention are CT55, DTX2P1-UPK3BP1-PMS2P11, EBF2, FAM173B and TMTC1, with the specific information shown in the following table:

| Inheritance Mode | Structural variation | Starting position | Ending position | Covering gene |
|---|---|---|---|---|
| X-linked inheritance | Insertion | 134294230 | 134346153 | CT55 |
| Autosomal recessive inheritance | Insertion | 76621574 | 76629524 | DTX2P1-UPK3BP1-PMS2P11 |
| | Insertion | 25862704 | 25867245 | EBF2 |
| | Deletion | 10227256 | 10232232 | FAM173B |
| | Deletion | 29839167 | 29850639 | TMTC1 |

For those skilled in the art, without departing from the scope of the technical solution of the present disclosure, many possible changes and modifications ban be made to the technical solutions of the present disclosure by using the technical contents disclosed above, or to modify the equivalent embodiments with equivalent changes. Therefore, any simple changes, equivalent changes and modifications based on the technical essence of the present disclosure without departing from the technical solution of the present disclosure should still fall into the protection scope of the technical solution of the present invention.

## Claims

1. A set of pathogenic genes of neuropsychiatric retardation and advanced cognitive disorder, comprising at least one of the following mutant genes: mutant genes of X-linked hereditary SLC16A2 and KIF4A, mutant genes of autosomal recessive hereditary MED15 and FAM83G, and five structural variant genes of CT55, DTX2P1-UPK3BP1-PMS2P11, EBF2, FAM173B and TMTC1.

2. The set of pathogenic genes of neuropsychiatric retardation and advanced cognitive disorder according to claim 1, wherein a cDNA coding region sequence of the SLC16A2 mutant gene is: the nucleotide G at position 1357 in exon region 5 of SLC16A2 gene sequence mutates into nucleotide C (c.G1357C).

3. The set of pathogenic genes of neuropsychiatric retardation and advanced cognitive disorder according to claim 1, wherein a cDNA coding region sequence of the KIF4A mutant gene is: the nucleotide C at position 1472 in exon region 14 of KIF4A gene sequence mutates into nucleotide T (c.C1472T).

4. The set of pathogenic genes of neuropsychiatric retardation and advanced cognitive disorder according to claim 1, wherein a cDNA coding region sequence of the MED15 mutant gene is: CAG nucleotides are inserted at position 573 in exon region 6 of the MED15 gene sequence (c.573insCAG).

5. The set of pathogenic genes of neuropsychiatric retardation and advanced cognitive disorder according to claim 1, wherein a cDNA coding region sequence of the FAM83G mutant gene is: ACC nucleotides are inserted at position 2455 in exon region 6 of the FAM83G gene sequence (c.2455insACC).

6. The set of pathogenic genes of neuropsychiatric retardation and advanced cognitive disorder according to claim 1, wherein the structural variant genes comprise five genes as follows:
the structural variant gene CT55d is insertion mutation, starting at position 134294230 and ending at position 134346153;
the structural variant gene DTX2P1-UPK3BP1-PMS2P11 is insertion mutation, starting at position 76621574 and ending at position 76629524;
the structural variant gene EBF2 is insertion mutation, starting at position 25862704 and ending at position 25867245;
the structural variant gene FAM173B is deletion mutation, starting at position 10227256 and ending at position 10232232;
the structural variant gene TMTC1 is deletion mutation, starting at position 29839167 and ending at position 29850639.

7. A detection primer for screening pathogenic genes of neuropsychiatric retardation and advanced cognitive disorder, comprising a PCR primer designed for mutant gene sequences in the set of pathogenic genes according to any one of claims 1 to 5, wherein the PCR primer sequence is selected from: primer sequences shown in SEQ ID NO. 5-6 for amplifying the SLC16A2 gene sequence, primer sequences shown in SEQ ID NO. 7-8 for amplifying the KIF4A gene sequence, primer sequences shown in SEQ ID NO.9-10 for amplifying the MED15 gene sequence, primer sequences shown in SEQ ID NO.11-12 for amplifying the FAM83G gene sequence.

8. A detection kit for screening pathogenic genes of neuropsychiatric retardation and advanced cognitive disorder, comprising the PCR primer sequence according to claim 7, PCR amplification enzyme and PCR amplification buffer.
